# EUROPEAN PATENT APPLICATION

(11) **EP 4 273 243 A1**
(43) Date of publication of application: **08.11.2023**
(21) Application number: 22382421.0
(22) Date of filing: 02.05.2022
(51) Int. Cl.: C12N 15/11, C12N 15/86, C12N 5/10, A61K 48/00

(54) **NUCLEIC ACID CONSTRUCTS AND VECTORS FOR PODOCYTE SPECIFIC EXPRESSION**

(71) Applicant: Fundació Hospital Universitari Vall d'Hebron - Institut de Recerca, 08035 Barcelona (ES); Universitat Autònoma de Barcelona, 08193 Cerdanyola del Vallès (Barcelona) (ES); Institució Catalana de Recerca I Estudis Avançats, 08010 Barcelona (ES)
(72) Inventor: Meseguer Navarro, Anna, 08023 Barcelona (ES); Chillon Rodriguez, Miguel, 08195 Barcelona (ES); Arévalo Bautista, Jazmine Paola, 08035 Barcelona (ES)
(74) Representative: Hoffmann Eitle

(57) **Abstract**

Present invention relates to a new podocyte-specific hybrid promoter and to gene constructs comprising it. The invention encompasses also new pharmaceutical compositions. All of them are for use as medicaments, in particular for use in the prevention and/or treatment of kidney diseases.

## Description

### Technical Field

Present invention relates to the field of selective expression of genes for gene therapy in podocytes of the kidney.

### Background Art

In the field of gene therapy, for example to substitute a mutated gene leading to a non-working or non-functional protein, one of the main concerns is to provide the copies of the genes to the cells (i.e., targets) of interest where the correct form is then to be expressed to perform its function.

Gene therapy is one of the approaches proposed for many diseases, such as in the case of many kidney diseases.

This targeting of the expression in the desired tissues or cells in a tissue, is usually performed with gene constructs (i.e., polynucleotide constructs) that comprise the gene of interest operatively linked with an expression promoter that is specific or mainly operative in the target cells.

One example of this is disclosed in the international patent application with publication number WO2020148548 (The University of Bristol), in which an adeno-associated virus vector comprising a nephrotic syndrome (NS) transgene under the control of the minimal nephrin promoter NPHS1 or under the control of podocin promoter NPHS2 is proposed. The aim of the authors was to achieve selectively the podocytes to perform there a gene therapy.

Thus, the gene constructs to be translated to functional proteins are usually embedded in expression vectors, such as in the previously commented adeno-associated viruses (AAV).

One of the main problems of the use of this expression vectors is that due to the elements that are needed to permit expression and first stability of the transcript (pre-mRNA or mRNA), the genes or fragments that can be loaded are those of low kilobases. Thus, in case the gene therapy aims to substitute a non-working gene of more than 1 kb approx., several complementary constructs (i.e., constructs prepared for trans-splicing and joining of the different fragments of a gene) are to be used, with the consequent complexity for the therapy.

In an attempt to introduce genes of higher kilobases one of the most used strategies is to use minimal promoters, and to reduce those number or types of regulatory elements in the AAV. However, this sometimes implies that expression is severely compromised. AAV vector genomes have been limited to 4.8 kilobases (kb) in length because their packaging limit. AAV vector genomes that exceeds 4.8 kb in length irrespective of the size of the plasmid-encoded vector or the capsid type show truncated genomes and severe manufacturing/production issues.

An example of another document disclosing a gene therapy for a kidney disease (Alport Syndrome) is disclosed in the international patent application with publication number WO2021181118A1 (The University of Bristol et al.). This document uses a construct with the minimal nephrin promoter (of 265 bp), with which the authors were able to successfully transduce human podocytes with full genes of more than 5 kb (i.e. COL4a3 and COL4a5). Although it is indicated that expression was in podocytes, nothing is stated regarding the expression being stimulated in damaged or non-functional podocytes. selectivity and the genome integrity of the packaged virus.

An additional aim of the gene therapy is further to target the defective cells within a group of cells of the same tissue. In the field of kidney diseases this is still an unmet need an, even in case for example expression can be predominant in podocytes, all podocytes express the transgene, meanwhile it would be desirable to stimulate at a higher extent the expression in these damaged or non-functional podocytes.

Thus, although many efforts have been carried out, there are still non-covered needs and the need of alternative methods for a successful and efficient gene therapy.

### Summary of Invention

Inventors surprisingly found that a fragment known as podocin gene enhancer (NPHS2 enhancer) that comprises NPHS2 Motif Lmx1b-FoxC2 in combination with a super core promoter including a TATA box motif (i.e., SCP1), allowed the selective expression of the genes of interest in podocytes and in kidney cell lines much more than other promoters of the prior art. Similar results are achievable with a core promoter, in particular minimal promoter derived from the cytomegalovirus promoter.

Hybrid promoters including enhancer elements and core regions are known, but their behaviour in terms of cell specificity and expression capability is not predictable.

Minimal promoters, even the hybrid ones, include generally elements that are not far from start codon of initiation of transcription.

Thus, a first aspect of the invention is a podocyte-specific hybrid promoter which comprises:
(a) a podocyte-specific enhancer sequence comprising SEQ ID NO: 13 (AAAAACAGAAGTTAGACCAGACCCCTTCCTGCCTATGATTCTTCAAGAAGCATTGCA TCATCAACATCAGGCATAAGCATTAATAAAGACCCTAAATAATAACAGAGACGAAACA CATCGCAAAGAGAGTTTTCTTTTATCCCCTTTAAAATGTAAATACTCCCAGGAGGAAT CAGCCAACATCATTAGGGGTTAATGCATATG), or a sequence with a percentage of identity with SEQ ID NO: 13 of at least 85%; and
(b) a core promoter sequence selected from:
   (b1) minimal core promoter of 81 nucleotides length with a percentage of identity from 85 to 100 % with SEQ ID NO: 2 (GTACTTATATAAGGGGGTGGGGGCGCGTTCGTCCTCAGTCGCGATCGAACACTCGA GCCGAGCAGACGTGCCTACGGACCG, and
   (b2) minimal core promoter of 56 nucleotides length with a percentage of identity from 85 to 100 % with SEQ ID NO: 20 (GGCGTTTACTATGGGAGGTCTATATAAGCAGAGCTCGTTTAGTGAACCGTCAGATC).

Indeed, this podocyte-specific hybrid promoter is one comprising:
(a) a podocyte-specific enhancer sequence, said enhancer sequence comprising from 200 to 250 nucleotides (or base pairs) isolated from the human coding podocin *(NPHS2)* gene promoter, said isolated sequence comprising SEQ ID NO: 7 (TTAATAAAGACCCTAAATA), which is a sequence that includes the binding motifs of the transcription factors Lmx1b and Foxc2 in the *NPHS2* gene promoter; and
(b) a core promoter sequence which TATA box motif is separated from last nucleotide in SEQ ID NO: 7 in the enhancer from 80 to 350 nucleotides (or base pairs), in particular from 123 to 126 nucleotides. SEQ ID NO: 7 is underwritten in SEQ ID NO: 13 in the previous paragraph.

Without being bound to any theory, inventors consider that the distance (in nucleotides or which is the same base pairs) between said SEQ ID NO: 7 and the TATA box is relevant for the leading of the expression; and that the selection of the particular cores promotes the expression in a selective way or at a higher extent in the podocytes, in relation to other hybrid promoters or naturally occurring promoters of the prior art.

A second aspect of the invention is a polynucleotide construct comprising the isolated podocyte-specific hybrid promoter as defined in the first aspect, and a nucleotide sequence of a gene or a fragment of a gene of interest (i.e., a polynucleotide of interest) operatively linked to the podocyte-specific hybrid promoter.

A third aspect of the invention is a vector comprising the podocyte-specific hybrid promoter as defined in the first aspect, or the polynucleotide construct as defined in the second aspect.

A fourth aspect of the invention is a cell comprising the podocyte-specific hybrid promoter as defined in the first aspect or the polynucleotide construct as defined in the second aspect, or the vector according to the third aspect.

In a fifth aspect the invention encompasses pharmaceutical compositions comprising a therapeutically effective amount of the polynucleotide construct according to the second aspect, the vector according to the third aspect or the cell according to the fourth aspect, together with one or more pharmaceutically acceptable excipients and/or carriers.

When the hybrid promoter, construct, or cell of the invention include a gene operatively linked to the promoter, said gene being a gene associated to a disease for example due to certain mutations, all the previous listed aspects can be used as a medicament, allowing the expression of a functional protein from said gene operatively linked to the hybrid promoter. Thus, in a sixth aspect, the invention relates to a promoter as defined in the first aspect, to a polynucleotide construct as defined in the second aspect, to a vector as defined in the third aspect, to a cell as defined in the fourth aspect, or to a pharmaceutical composition as defined in the fifth aspect for use as a medicament.

### Brief Description of Drawings

FIG. 1, related with example 1 shows a scheme of polynucleotide constructs according to the invention and also from the prior art, all containing the gene of the green fluorescent protein (GFP).
FIG. 2, related with example 1, illustrates the fluorescence in a confocal microscope emitted by the green fluorescent protein (GFP) in transduced cells with the constructs of FIG. 1.
FIG. 3, related with Example 1, depicts the expression of Lmx1b and Foxc2 transcription factors in the selected cell lines.
FIG. 4, related with example 2, is a schematic view of the promoters and luciferase gene to determine the expression of this protein in podocytes (relative luciferase units (RLU) in the podocytes for each of the assayed constructs).
FIG. 5, related with example 3, shows a scheme of the elements (in boxes) of the constructs of the invention (with hybrid promoter of SEQ ID NO. 1) differing in the polyadenylation signal (i.e., SV40polyA or CPEpolyA). The figure illustrates the expression in HEK293 cells under endoplasmic reticulum stress conditions (ER stress). Data including protein expressions (relative luciferase expression) and mRNA levels (relative mRNA expression) are provided along time of ER stress.
FIG. 6 is a graphic with the bars of the relative expression of the Crumbs Cell Polarity Complex Component 2 gene (CRB2, UniProtKB/Swiss-Prot: Q5IJ48, Sequence version 2 (17 Oct 2006)) in HEK293 cells using a polynucleotide construct with the promoter of SEQ ID NO: 1 in comparison with a construct carrying the cytomegalovirus promoter (CMV).

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the," also include the plural of the noun.

The term "promoter" refers to a sequence of DNA to which certain proteins bind allowing initiating the transcription of a single RNA from the DNA downstream of it. There are three main portions that make up a promoter: core promoter, proximal promoter, and distal promoter. The core promoter region is located most proximal to the start codon and contains the RNA polymerase binding site, TATA box, and transcription start site (TSS). RNA polymerase will bind to this core promoter region stably and transcription of the template strand can initiate. The TATA box is a DNA sequence (5'-TATAAA-3') within the core promoter region where general transcription factor proteins and histones can bind. Histones are proteins found in eukaryotic cells that package DNA into nucleosomes. Histone binding prevents the initiation of transcription whereas transcription factors promote the initiation of transcription. The most 3' portion (closest to the gene's start codon) of the core promoter is the TSS which is where transcription actually begins. Only eukaryotes and archaea, however, contain this TATA box. Most prokaryotes contain a sequence thought to be functionally equivalent called the Pribnow box which usually consists of the six nucleotides, TATAAT. Further upstream from the core promoter one can find the proximal promoter which contains many primary regulatory elements. The proximal promoter is found approximately 250 base pairs upstream from the TSS and it is the site where general transcription factors bind. The final portion of the promoter region is called the distal promoter which is upstream of the proximal promoter. The distal promoter also contains transcription factor binding sites, but mostly contains regulatory elements.

The term "minimal promoter" refers to a minimal sequence of a native promoter (mostly core promoter) that could express a downstream gene.

The expression "hybrid promoter" is to be understood as a combination of elements derived from different promoters (i.e., naturally occurring promoters). For example, a hybrid promoter may comprise a proximal or distal promoter region derived from one pre-existing promoter and a core promoter from another pre-existing promoter to achieve the desired transgene expression.

The "enhancers or enhancer sequences" are short (15-1500 bp) region of DNA that can be bound by proteins (activators) to increase the likelihood that transcription of a particular gene will occur. These proteins are usually referred to as transcription factors. Enhancers are cis-acting. They can be located up to 1 Mbp (1,000,000 bp) away from the gene, upstream or downstream from the start site.

When sequences of polynucleotides are indicated, they refer to informative molecules and only one strand is shown. Nonetheless, the skilled person in the art will understand that they can be in form of single or double stranded nucleic acid polymers, for example in case of the desoxyribonucleic acid including a template and the complementary strand, or a single strand ribonucleic acid, for example the messenger RNA. The sequences of polynucleotides indicated in this description are either disclosed in terms of their length in nucleotides or in terms of bases, nucleobases or their multiples (e.g., kilobases, kb), which are the same, since the bases are part of the nucleotides. When the expression "base pairs" is used, then it relates to the fundamental unit of double-stranded nucleic acids consisting of two nucleobases bound to each other by hydrogen bonds. The number of base pairs is another equivalent way to indicate the length of a polynucleotide when referring to it as a double-stranded molecule.

As previously indicated, a first aspect of the invention is a podocyte-specific hybrid promoter which comprises:
(a) a podocyte-specific enhancer sequence comprising SEQ ID NO: 13 (AAAAACAGAAGTTAGACCAGACCCCTTCCTGCCTATGATTCTTCAAGAAGCATTGCA TCATCAACATCAGGCATAAGCATTAATAAAGACCCTAAATAATAACAGAGACGAAACA CATCGCAAAGAGAGTTTTCTTTTATCCCCTTTAAAATGTAAATACTCCCAGGAGGAAT CAGCCAACATCATTAGGGGTTAATGCATATG), or a sequence with a percentage of identity with SEQ ID NO: 13: of at least 85%; and
(b) a core promoter sequence selected from:
   (b1) minimal core promoter of 81 nucleotides length with a percentage of identity from 85 to 100 % with SEQ ID NO: 2 (GTACTTATATAAGGGGGTGGGGGCGCGTTCGTCCTCAGTCGCGATCGAACACTCGA GCCGAGCAGACGTGCCTACGGACCG, and
   (b2) minimal core promoter of 56 nucleotides length with a percentage of identity from 85 to 100 % with SEQ ID NO: 20 (GGCGTTTACTATGGGAGGTCTATATAAGCAGAGCTCGTTTAGTGAACCGTCAGATC).

Indeed, this podocyte-specific hybrid promoter is one comprising:
(a) a podocyte-specific enhancer sequence, said enhancer sequence comprising from 200 to 250 nucleotides (or base pairs) isolated from the human coding podocin *(NPHS2)* gene promoter, said isolated sequence comprising SEQ ID NO: 7 (TTAATAAAGACCCTAAATA), which is a sequence that includes the binding motifs of the transcription factors Lmx1b and Foxc2 in the *NPHS2* gene promoter; and
(b) a core promoter sequence which TATA box motif is separated from last nucleotide in SEQ ID NO: 7 in the enhancer from 80 to 350 nucleotides (or base pairs), in particular from 123 to 126 nucleotides. SEQ ID NO: 7 is underwritten in SEQ ID NO: 13 in the previous paragraph.

In a particular embodiment of the podocyte-specific hybrid promoter, the podocyte-specific enhancer sequence is a sequence of 219 nucleotides length with a % of identity from 85 to 100 % with SEQ ID NO: 3. This SEQ ID NO: 3 comprises SEQ ID NO: 13.

In a particular embodiment of the podocyte-specific hybrid promoter, it comprises:
(a) a podocyte-specific enhancer sequence comprising or consisting of SEQ ID NO: 3; and
(b) minimal core promoter of 81 nucleotides length with a % of identity from 85 to 100 % with SEQ ID NO:2 (SCP1)

(GTACTTATATAAGGGGGTGGGGGCGCGTTCGTCCTCAGTCGCGATCGAACACTCGA
GCCGAGCAGACGTGCCTACGGACCG).

In a more particular embodiment, the podocyte-specific promoter comprises or consists of SEQ ID NO: 1, or in a nucleotide sequence with a percentage of identity of at least 85 % with said SEQ ID NO: 1. 
AAAAACAGAAGTTAGACCAGACCCCTTCCTGCCTATGATTCTTCAAGAAGCATTGCAT
CATCAACATCAGGCATAAGCATTAATAAAGACCCTAAATAATAACAGAGACGAAACAC
ATCGCAAAGAGAGTTTTCTTTTATCCCCTTTAAAATGTAAATACTCCCAGGAGGAATC
AGCCAACATCATTAGGGGTTAATGCATATGTAGAATAACTAGGGCGTACTTATATAAG
GGGGTGGGGGCGCGTTCGTCCTCAGTCGCGATCGAACACTCGAGCCGAGCAGACG
TGCCTACGGACCG
 (SEQ ID NO. 1 is also abbreviated in this description as 0.3NPHS2-SCP1 - 300 bp)

This isolated podocyte-specific hybrid promoter can also be defined as a polynucleotide with a length from 300 to 330 nucleotides (or base pairs), which comprises or consists in SEQ ID NO: 1 or a sequence with a percentage of identity of at least 85% with this SEQ ID NO: 1.

From a detailed analysis of this SEQ ID NO: 1, the podocyte-specific hybrid promoter comprises a polynucleotide sequence comprising:
- A minimal core promoter of 81 nucleotides length with a % of identity from 85 to 100 % with of SEQ ID NO: 2 (GTACTTATATAAGGGGGTGGGGGCGCGTTCGTCCTCAGTCGCGATCGAACA CTCGAGCCGAGCAGACGTGCCTACGGACCG); and
- A podocyte-specific enhancer of 219 nucleotides length with a % of identity from 85 to 100 % with SEQ ID NO: 3

(AAAAACAGAAGTTAGACCAGACCCCTTCCTGCCTATGATTCTTCAAGAAGCA
TTGCATCATCAACATCAGGCATAAGCATTAATAAAGACCCTAAATAATAACAGA
GACGAAACACATCGCAAAGAGAGTTTTCTTTTATCCCCTTTAAAATGTAAATAC
TCCCAGGAGGAATCAGCCAACATCATTAGGGGTTAATGCATATGTAGAATAAC
TAGGGC)

In this SEQ ID NO: 1, from nucleotide 1 to 219 [1 : 219], corresponds to the NPHS2 enhancer; from nucleotide 80 to 98 [80 : 98] corresponds to the motif Lmx1b-FoxC2 (i.e., where the transcription factors are bounded, underwritten); the minimal promoter known as super core minimal promoter 1 (SCP1) extends from nucleotide 220 to 300 [220 : 300], and TATA box in this minimal promoter is located from nucleotide 225 to 231 [225 : 231] (double-underwritten). SCP1 is widely defined in Juven-Gershon et al., "Rational design of super core promoter that enhances gene expression", Nature Methods-2006, vol. no. 3(11), pp. 917-922.

In yet another particular embodiment, the podocyte-specific hybrid promoter comprises:
(a) a podocyte-specific enhancer sequence comprising or consisting of SEQ ID NO: 13 (AAAAACAGAAGTTAGACCAGACCCCTTCCTGCCTATGATTCTTCAAGAAGCATTGCA TCATCAACATCAGGCATAAGCATTAATAAAGACCCTAAATAATAACAGAGACGAAACA CATCGCAAAGAGAGTTTTCTTTTATCCCCTTTAAAATGTAAATACTCCCAGGAGGAAT CAGCCAACATCATTAGGGGTTAATGCATATG) and
(b) a minimal core promoter sequence of 56 nucleotides length with a % of identity from 85 to 100 % with SEQ ID NO: 20 (GGCGTTTACTATGGGAGGTCTATATAAGCAGAGCTCGTTTAGTGAACCGTCAGATC), more in particular consisting of SEQ ID NO: 20.

In a more particular embodiment, the podocyte-specific promoter comprises or consists of SEQ ID NO: 4 [also labelled in this description and figures as 0.2NPH2+minCMV]

(AAAAACAGAAGTTAGACCAGACCCCTTCCTGCCTATGATTCTTCAAGAAGCATTGCA
TCATCAACATCAGGCATAAGCATTAATAAAGACCCTAAATAATAACAGAGACGAAACA
CATCGCAAAGAGAGTTTTCTTTTATCCCCTTTAAAATGTAAATACTCCCAGGAGGAAT
CAGCCAACATCATTAGGGGTTAATGCATATGGGCGTTTACTATGGGAGGTCTATATAA
GCAGAGCTCGTTTAGTGAACCGTCAGATC)

Alternatively, the hybrid promoter of the first aspect can be defined by the subsequences with an identified function composing it (see above).

The sequence defining the promoter of the first aspect (e.g., SEQ ID NO:1, SEQ ID NO: 4 or a sequence with at least a percentage of identity of 85 % with the same, or any of the previously disclosed in the embodiments) is the promoter sequence of a polynucleotide construct comprising operatively linked thereto the sequence of a gene of interest or fragment thereof, or the sequence of a polynucleotide of interest. Thus, as also indicated, a second aspect of the invention is a polynucleotide construct comprising the isolated podocyte-specific hybrid promoter as defined in the first aspect, and a nucleotide sequence of a gene or a fragment of a gene of interest operatively linked to the podocyte-specific hybrid promoter.

Protein variants are well understood to those of skill in the art and can involve amino acid sequence modifications derived from nucleotide modifications. For example, amino acid sequence modifications typically fall into one or more of three classes: substitutional, insertional, or deletional variants. In the same way nucleotide sequence variants, independently of their stablished role (i.e., promoters of genes, codifying gene sequences, etc.) can also involve nucleotide sequence modifications including deletions, insertions, changes of nucleotides.

In the present invention, the term "identity" refers to the percentage of residues that are identical in the two sequences when the sequences (either of amino acid or of nucleic acid) are optimally aligned. If, in the optimal alignment, a position in a first sequence is occupied by the same amino acid or nucleotide residue as the corresponding position in the second sequence, the sequences exhibit identity with respect to that position. The percentage of identity determines the number of identical residues over a defined length in a given alignment. Thus, the level of identity between two sequences or ("percent sequence identity") is measured as a ratio of the number of identical positions shared by the sequences with respect to the number of positions compared (i.e., percent sequence identity = (number of identical positions/total number of positions compared) x 100). A gap, i.e., a position in an alignment where a residue is present in one sequence but not in the other, is regarded as a position with non-identical residues and is counted as a compared position.

A number of mathematical algorithms for rapidly obtaining the optimal alignment and calculating identity between two or more sequences are known and incorporated into a number of available software programs. For purposes of the present invention, the sequence identity between two amino acid sequences or two nucleotide sequences is preferably determined using algorithms based on global alignment, such as the Needleman-Wunsch algorithm (Needleman and Wunsch, 1970, J. Mol. Biol. 48: 443-453), preferably implemented in the Needle program of the EMBOSS package (EMBOSS: The European Molecular Biology Open Software Suite, Rice et al., 2000, Trends Genet. 16: 276-277); or the BLAST Global Alignment tool (Altschul et al., "Basic local alignment search tool", 1990, J. Mol. Biol, v. 215, pages 403-410), using default settings. Local alignment also can be used when the sequences being compared are substantially the same length.

In a particular embodiment of the first aspect of the invention, optionally in combination with any one of the embodiments provided below, the percentages of identity with any of the therein identified sequences (i.e., enhancer, core promoter) are selected from at least 85%, at least 86%, at least 87%, at least 88%, at least 88.5%, at least 89%, at least 89.5%, at least 90%, at least 90.5%, at least 91%, at least 91.5%, at least 92%, at least 92.5%, at least 93%, at least 93.5%, at least 94%, at least 94.5%, at least 95%, at least 95.5%, at least 96%, at least 96.5%, at least 97%, at least 97.5%, at least 98%, at least 98.5%, at least 99%, or at least 99.5% identical to the indicated sequences.

In a particular embodiment of the first aspect of the invention, optionally in combination with any one of the embodiments provided below, the podocyte-specific hybrid promoter consists of sequence SEQ ID NO: 1 or a functional variant thereof, said variant consisting of a sequence at least 85%, 86%, 87%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5% identical to SEQ ID NO: 1.

Herewith disclosed is a podocyte-specific hybrid promoter that consists of sequence SEQ ID NO: 4 or a functional variant thereof consisting of a sequence at least 85%, 86%, 87%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5% identical to SEQ ID NO: 4.

The term "nucleic acid construct" or "polynucleotide construct" refers to a man-made nucleic acid molecule resulting from the use of recombinant DNA technology. A nucleic acid construct is a nucleic acid molecule, either single- or double-stranded, which has been modified to contain segments of nucleic acids, which are combined and juxtaposed in a manner, which would not otherwise exist in nature. A nucleic acid construct usually is a "vector", i.e. a nucleic acid molecule which is used to deliver exogenously created DNA into a host cell. It can also be named as a "gene construct" or "expression cassette", when it refers to a polynucleotide sequence including in turn a sequence coding for a protein of interest, which is operatively linked to an expression promoter, said promoter controlling expression of the sequence coding for the protein. For "operatively linked" is to be understood that the sequence coding for the protein is disposed after the sequence of the promoter (in the 5'-3' direction), or near the promoter in case restriction sites are included, or other stabilizing elements of the gene construction are present. The expression cassette itself is also a expression system, being vectors or plasmids further used to protect the gene construct, or to promote entrance to cells in case of viral vectors. For "polynucleotide sequence" it is to be understood as a nucleic acid molecule (DNA or RNA) comprising deoxyribonucleotides or ribonucleotides. Nucleic acid can be single or double stranded, and it includes, but it is not limited to, nucleotide sequences coding for polypeptides.

In a particular embodiment of the second aspect, this polynucleotide construct further comprises one or more of:
(a) a polyadenylation signal nucleotide sequence;
(b) a protein translation initiation site consensus nucleotide sequence;
(c) a post-transcriptional regulatory element nucleotide sequence; and
(d) 5' and 3' inverted terminal repeat nucleotide sequences.

All these elements (a) to (d) are sequences that allow the proper expression of the gene operatively linked to a promoter.

In a particular embodiment of the second aspect, the polynucleotide construct comprises the podocyte-specific hybrid promoter as defined in the first aspect, and a nucleotide sequence of a gene or a fragment of a gene of interest operatively linked to the podocyte-specific hybrid promoter, and one or more of: a polyadenylation signal nucleotide sequence; a protein translation initiation site consensus nucleotide sequence; and a post-transcriptional regulatory element nucleotide sequence.

The polyadenylation signal is a sequence that facilitates the transcription by stabilizing the transcript mRNA that is further going to be translated. Polyadenylation is the addition of a poly(A) tail to a messenger RNA. The poly(A) tail consists of multiple adenosine monophosphates; in other words, it is a stretch of RNA that has only adenine bases. The poly(A) tail is important for the nuclear export, translation, and stability of mRNA. Inclusion of a polyadenylation signal can therefore enhance expression of the gene of interest.

The protein translation initiation site is a nucleotide sequence that facilitates initiation of translation by mediating ribosome assembly and translation initiation.

The post-transcriptional regulatory elements are sequences that enhance virus (i.e., vector) stability in packaging cells, leading to higher titre of packaged virus, hence, expression of transgenes (i.e., genes of interest operatively linked to the promoter).

The 5' and 3' inverted terminal repeat (5'-ITR and 3'-ITR) are regions essentially identical in sequence that reside on both ends of an adeno-associated virus genome pointing opposite directions serving as the origin of viral genome replication. ITR are usually flanking the desired gene together with the promoter, and they aid in concatemer formation in the nucleus after the single-stranded vector DNA is converted by host cell DNA polymerase complexes into double-stranded DNA. The skilled person in the art will know which kind of sequences work as 5'- and 3'-ITR.

In a more particular embodiment of the second aspect, the polynucleotide construct comprises in the 5' to 3' direction:
(i) a 5' inverted terminal repeat nucleotide sequence;
(ii) the isolated polynucleotide hybrid promoter as defined in the first aspect (claim 1);
(iii) a protein translation initiation site consensus nucleotide sequence;
(iv) a nucleotide sequence of a gene or a fragment of a gene of interest;
(v) a post-transcriptional regulatory element nucleotide sequence;
(vi) a polyadenylation signal nucleotide sequence; and
(vii) a 3' inverted terminal repeat nucleotide sequence.

In a more particular embodiment of the polynucleotide construct according to the second aspect, the protein translation initiation site consensus nucleotide sequence is the kozak consensus sequence. The Kozak sequence is known to play a major role in the initiation of the translation process and can therefore enhance expression of the gene of interest. The Kozak consensus sequence (Kozak consensus or Kozak sequence) is a nucleic acid motif that functions as the protein translation initiation site in most eukaryotic mRNA transcripts.

In another particular embodiment of the polynucleotide construct, optionally in combination with any of the embodiments above or below of this polynucleotide construct, the post-transcriptional regulatory element nucleotide sequence is a Woodchuck hepatitis postranscription regulatory element (WPRE). WPRE is a DNA sequence that, when transcribed, creates a tertiary structure enhancing expression. Inclusion of WPRE elements may increase expression of the transgene delivered by a vector, when this polynucleotide construct is in a vector. In a more particular embodiment, WPRE is the WPRE3 of SEQ ID NO: 12 (GATAATCAACCTCTGGATTACAAAATTTGTGAAAGATTGACTGGTATTCTTAACTATG TTGCTCCTTTTACGCTATGTGGATACGCTGCTTTAATGCCTTTGTATCATGCTATTGCT TCCCGTATGGCTTTCATTTTCTCCTCCTTGTATAAATCCTGGTTAGTTCTTGCCACGG CGGAACTCATCGCCGCCTGCCTTGCCCGCTGCTGGACAGGGGCTCGGCTGTTGGG CACTGACAATTCCGTGGT). WPRE3 is shorter than WPRE. WPRE comprises 598 base pairs, and WPRE3 comprises 248 base pairs, 248 bp but it is similarly efficient on increasing the expression of the transgene.

In another particular embodiment of the polynucleotide construct, optionally in combination with any of the embodiments above or below of this polynucleotide construct, the polyadenylation signal nucleotide sequence is selected from:
(i) bovine growth hormone (bGH) polyadenylation signal,
(ii) early SV40 polyadenylation signal,
(iii) synthetic polyadenylation signal (SpA), and
(iv) an untranslated sequence in 3' position (3'-UTR) with respect to the polynucleotide sequence of a gene or a fragment of a gene of interest that comprises at least two cytoplasmic polyadenylation elements (CPE) which are separated by less than 50 nucleotides, and a cytoplasmic polyadenylation signal which is separated by less than 100 nucleotides from the first or second cytoplasmic polyadenylation element.

In a more particular embodiment of the polynucleotide construct, optionally in combination with any of the embodiments above or below of this polynucleotide construct, the polyadenylation signal nucleotide sequence is an untranslated sequence that comprises at least two cytoplasmic polyadenylation elements which are separated by less than 50 nucleotides, and a cytoplasmic polyadenylation signal which is separated by less than 100 nucleotides from the first or second cytoplasmic polyadenylation.

The term "cytoplasmic polyadenylation element" or "CPE", as used herein, refers to a sequence which is found in the 3'UTR of messenger RNAs. CPEs usually have the sequence 5'-UUUUAi_2 U-3' (also called consensus sequence), although other variants are possible, for example, non-consensus sequences: UUUUACU, UUUCAU and UUUUCCU. CPE are bound by CPE binding proteins (CPEBs), which promotes the extension of the existing polyadenylation tail and, in general, the translation of the mRNA. The skilled person can determine if a particular sequence is a CPE by the method described by Pique et al in "A Combinatorial Code for CPE-Mediated Translational Control" Cell 2008, 132(3): 434-48.

In a particular embodiment, the first CPE has the consensus sequence 5'- UUUUAi_2 U-3' when the nucleic acid construct is an RNA molecule. When the nucleic acid construct is a DNA molecule, then the CPE comprises a sequence which, when transcribed, results in the above sequence".

In a particular embodiment, the second CPE has the consensus sequence 5'- UUUUAi_2 U-3' when the nucleic acid construct is an RNA molecule. When the nucleic acid construct is a DNA molecule, then the CPE comprises the sequence which, when transcribed, results in the above sequence.

In a particular embodiment, the first and second CPE has the consensus sequences 5'-UUUUAi_2 U-3' when the nucleic acid construct is an RNA molecule. When the nucleic acid construct is a DNA molecule, then the CPE comprises the sequence which, when transcribed, results in the above sequence.

In a more particular embodiment, the first CPE has the consensus sequence 5'-UUUUUAA-3'.

In a more particular embodiment, the second CPE has the consensus sequence 5'-UUUUAAU-3'.

In an even more particular embodiment, the first CPE has the consensus sequence 5'-UUUUUAA-3' and the second CPE has the consensus sequence 5'- UUUUAAU-3' when the nucleic acid construct is a RNA molecule or, when the nucleic acid construct is a DNA molecule, a sequence that, when transcribed, results in the above sequence.

In a particular embodiment, the 3'UTR of the polynucleotide of the invention further comprises a third CPE. In a more particular embodiment, the third CPE has a non-consensus sequence. In an even more particular embodiment, the third CPE has the non-consensus sequence 5'-UUUUACU-3' when the nucleic acid construct is an RNA molecule or, when the nucleic acid construct is a DNA molecule, a sequence that, when transcribed, results in the above sequence.

In a particular embodiment, the third CPE is located in a 3' position with respect to the first CPE but in a 5' position with respect to the cytoplasmic polyadenylation signal. In another particular embodiment, the third CPE is located in a 5' position with respect to the first CPE and to the cytoplasmic polyadenylation signal. In another particular embodiment, the third CPE is located between the first and second CPE and in a 3' position with respect to the cytoplasmic polyadenylation signal.

In a particular embodiment, the first CPE has the consensus sequence 5'-UUUUUAA-3', the second CPE has the consensus sequence 5'-UUUUAAU-3' and the 3'UTR further comprises a third CPE with the non-consensus sequence 5'-UUUUACU- 3' when the nucleic acid construct is an RNA molecule or, when the nucleic acid construct is a DNA molecule, a sequence that, when transcribed, results in the above sequence.

When the nucleic acid construct is an RNA molecule, said nucleic acid construct further comprises a polyadenine tail.

The 3'UTR of the nucleic acid construct of the first aspect comprises a cytoplasmic polyadenylation signal which is separated by less than 100 nucleotides from the second CPE. The term "cytoplasmic polyadenylation signal", as used herein, refers to a nucleic acid sequence which is present in the 3'UTR of mRNA and promotes the cytoplasmic polyadenlylation of the mRNA. In a particular embodiment, the cytoplasmic polyadenylation signal is the hexanucleotide AAUAAA.

The expression "separated by less than 100 nucleotides from the first or second CPE" means that the number of nucleotides between the last nucleotide of the first or second CPE and the first nucleotide of the cytoplasmic polyadenylation signal is less than 100 nucleotides. In a particular embodiment, the number of nucleotides between the first or second CPE and the cytoplasmic polyadenylation signal is less than 90, less than 80, less than 70, less than 60, less than 50, less than 40, less than 30, less than 20, less than 10, less than 5 nucleotides. In a more particular embodiment, the distance between the first or second CPE and the cytoplasmic polyadenylation signal is 25 nucleotides. In particular embodiment, there is no nucleotide between the second CPE and the cytoplasmic polyadenylation signal. In another particular embodiment, the second CPE and the cytoplasmic polyadenylation signal partially overlap.

In a particular embodiment, the cytoplasmic polyadenylation signal is located in a 3' position with respect to the first and second CPE.

In another embodiment, the second cytoplasmic polyadenylation element can overlap with the cytoplasmic polyadenylation signal. In a particular embodiment, the second CPE overlaps at least one, at least 2, at least 3, at least 4, at least 5, or at least 6 nucleotides with the cytoplasmic polyadenylation signal.

In another particular embodiment, when the 3 'UTR comprises a third CPE, the cytoplasmic polyadenlylation signal is located in a 3' position with respect to the third CPE.

These particular embodiments of the polyadenylation signal including CPE elements and a cytoplasmic polyadenylation signal were widely commented in the international application with publication number WO2017153606 (Fundació Institut de Recerca Biomèdica (IRB Barcelona), et al.). The document proposes these 3'UTR sequences in a construct to selectively express genes of interest in cancer cells. However, the behavior of tumor cells is not directly transposable to any other diseased cell (i.e., kidney), and there is also bibliography with contradictory results.

As will be depicted in the examples, the combination of the particular indicated hybrid promoter with the polyadenylation signal including CPE elements allows to express a desired gene therapy transgene in all the damaged podocytes in a higher extent (i.e., stimulated). When the gene therapy expression is able to restore a healthier state of podocytes, then gene therapy transgene expression will still be expressed at basal levels, Thus, the transgene is expressed in all the podocytes, and favourably at a higher extend in the damaged ones.

In another particular embodiment of the polynucleotide construct, optionally in combination with any of the embodiments above or blow of this polynucleotide construct, the gene or fragment thereof of interest operatively linked to the podocyte-specific hybrid promoter is selected from:
- a gene associated with one or more kidney diseases, and/or
- a gene with a length up to 5 kb, more in particular up to 4.5 kb, and also in particular up to 4.0 kb.

In a more particular embodiment, the genes or fragments thereof are selected from one or more of Crumbs Cell Polarity Complex Component 2 gene (CRB2), Nephrin gene *(NPHS1);* Podocin gene *(NPHS2);* aarF domain containing kinase 4 *(ADCK4)* gene; Chitobiosyldiphosphodolichol Beta-Mannosyltransferase gene *(ALG1);* Rho GTPase Activating Protein 24 gene *(ARHGAP24); ARGHDIA CD151* - *CD2AP;* Coenzyme Q2, Polyprenyltransferase gene (COQ2); Coenzyme Q6, Monooxygenase gene (COQ6); Diacylglycerol Kinase Epsilon gene *(DGKE);* E2F Transcription Factor 3 gene (*E2F3*)*;* Epithelial Membrane Protein 2 gene (*EMP2*)*;* KN Motif And Ankyrin Repeat Domains 2 gene (*KANK2*)*;* L Antigen Family Member 3 gene (*LAGE3*)*;* LMNA gene *(LMNA)*; LIM homeobox transcription factor 1-beta gene *(LMX1B);* Transcription factor MafB gene *(MAFB);* Nucleoporin 85 gene (*NUP85*); Nucleoporin 93 gene (*NUP93*); Nuclear RNA Export Factor 5 gene (*NXF5);* O-Sialoglycoprotein Endopeptidase gene (OSGEP); Paired box genes *(PAX);* Decaprenyl Diphosphate Synthase Subunit 2 gene (*PDSS2*)*;* phosphomannomutase 2 gene (*PMM2);* Podocalyxin Like gene *(PODXL);* Scavenger Receptor Class B Member 2 gene (SCARB2); sphingosine-1-phosphate lyase 1 gene *(SGPL1);* SMAD Family Member 7 gene (*Smad7*)*;* TP53 Regulating Kinase gene (TP53RK); TP53RK Binding Protein gene *(TPRKB);* Vitamin D Receptor gene *(VDR);* WD Repeat Domain 73 gene (*WDR73);* Wilms' tumor 1 gene *(WT1);* zinc metallopeptidase gene (*ZMPSTE2);* Apolipoprotein L1 gene *(APOL1),* Collagen Type IV Alpha 3 Chain gene(*COL4A3);* Collagen Type IV Alpha 4 Chain gene (COL4A4), Collagen Type IV Alpha 5 Chain gene (COL4A5), Collagen Type IV Alpha 6 Chain gene (*COL4A6),* Klotho protein gene *(Klotho),* Fibroblast Growth Factor 23 gene (*FGF23),* Bone Morphogenetic Protein 7 gene (BMP7),and vascular endothelial growth factor *(VEGF)* C gene. All these genes are, in a particular embodiment when included in the construct of the invention, in form or minigenes. Thus, they are gene fragments that include one or more exons and the control regions necessary for the gene to express itself in the same way as a wild type gene.

In a particular embodiment, the polynucleotide construct is selected from the group consisting of:
SEQ ID NO: 6, or a functionally equivalent variant with at least 85% of identity with SEQ ID NO: 6.
SEQ ID NO: 18, or a functionally equivalent variant with at least 85% of identity with SEQ ID NO: 18 (also labelled in this description as 0.3NPHS2-SCP1_Kco.hCRB2_minSV40pA) SEQ ID NO: 19, or a functionally equivalent variant with at least 85% of identity with SEQ ID NO: 19.

In another particular embodiment, the polynucleotide construct is selected from the group consisting of:
SEQ ID NO: 8, or a functionally equivalent variant with at least 85% of identity with SEQ ID NO: 8 (0.3_Luc_minSV40 [2375 bp] MiniHybrid NTX, see Example 2)
SEQ ID NO: 9, or a functionally equivalent variant with at least 85% of identity with SEQ ID NO: 9 (0.3_Luc_CPE [2309 bp, 0.3_Luc_CPE [2309 bp], see Example 3)
SEQ ID NO: 10, or a functionally equivalent variant with at least 85% of identity with SEQ ID NO: 10 (0.3NPHS2-SCP1_KTurboGFP_WPRE3_minSV40pA, see Example 1)
SEQ ID NO: 11, (0.2NPHS2-minCMV_KTurboGFP_WPRE3_minSV40pA, see Example 1).

In a third aspect, the invention relates to a vector comprising the podocyte-specific hybrid promoter as defined in the first aspect or the polynucleotide construct as defined in the second aspect.

The term "vector", as used herein, refers to a construct capable of delivering, and preferably additionally expressing, one or more polynucleotides of interest into a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. This term also relates to targeting constructs which allow for random or site-directed integration of the targeting construct into genomic DNA. Such targeting constructs, preferably, comprise DNA of sufficient length for either homologous recombination or heterologous integration In a particular embodiment of the vector, this is an expression vector.

The term "expression vector" refers to a replicative DNA construct used for expressing the nucleic acid construct of the invention in a cell, preferably a eukaryotic cell, more preferably a mammalian cell. Expression vectors are the basic tools in biotechnology for the production of proteins. An expression vector has features that any vector may have, such as an origin of replication, a selectable marker, and a suitable site for the insertion of a gene such as the multiple cloning site. The expression vector also preferably contains an origin of replication in prokaryotes, necessary for vector propagation in bacteria. Additionally, the expression vector can also contain a selection gene for bacteria, for example, a gene encoding a protein conferring resistance to an antibiotic, for example, ampicillin, kanamycin, chloramphenicol, etc. The expression vector can also contain one or more multiple cloning sites.

In even a more particular embodiment of the vector of the invention, it is an adeno-associated virus vector (AAV), in particular selected from one or more of the serotypes, AAV1, AAV2, AAV3B, AAV4, AAV8, AAV9, AAVrh10, AAVLK03, AAV-DJ, and AAV-DJ/8. In particular, any other AAV with tropism for kidney tissues, and more in particular for podocyte or tubule cells.

The term "adeno-associated virus (AAV)" as used herein refers to a viral vector that infects both dividing and quiescent primate (and human) cells. Because they seem to lack any pathogenic effects, and usually integrate in the same place of the genome (the AAVS1 site, in chromosome 19), this viral vectors can safely be used to transduce foreign DNA into human cells in gene therapy applications. Generally, the AAV serotypes have genomic sequences of significant homology at the amino acid and the nucleic acid levels, provide an identical set of genetic functions, produce virions which are essentially physically and functionally equivalent, and can be engineered to express transgenes that require tissue-specific regulation.

A fourth aspect of the invention is a cell comprising the podocyte-specific hybrid promoter as defined in the first aspect, or the polynucleotide construct as defined in the second aspect, or the vector according to the third aspect. The cell includes one or more of these listed elements in such a mode that they are functional, i.e., they allow expression of the gene or fragment of interest.

In a particular embodiment, the cell is an animal cell, more in particular a mammal cell, and even more in particular a human cell.

The method for the synthesis of the constructs, vectors, as well as all the molecular biology tools for the cell transfection or transduction are hose well-known by the skilled person in the art and duly established in the protocols for molecular biology. Particular conditions are indicated in the examples.

The invention also encompasses pharmaceutical compositions comprising a therapeutically effective amount of any of the polynucleotide construct according to the invention, the vector according to the invention, or the cells according to the invention, together with one or more pharmaceutically acceptable excipients and/or carriers.

The term "pharmaceutical" encompasses also the concept of "veterinary composition". Thus, they relate to compositions that are therapeutically effective when administered by any desired or applicable route to any animal, including humans.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio. Examples of suitable acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Excipients such as coloring agents, coating agents, sweetening, and flavoring agents can be present in the composition, according to the judgment of the formulator.

The pharmaceutical compositions containing the polynucleotide construct according to the invention, the vector according to the invention, or the cells of the invention, can be presented in any dosage form, for example, solid or liquid, and can be administered by any suitable route, for example, oral, parenteral, rectal, topical, intranasal, intraocular, intraperitoneal or sublingual route, for which they will include the pharmaceutically acceptable excipients necessary for the formulation of the desired dosage form, for example, topical formulations (ointment, creams, lipogel, hydrogel, etc.), eye drops, aerosol sprays, injectable hydrogels, injectable solutions, osmotic pumps, etc.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, corn-starch, powdered sugar, and combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked polyvinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and combinations thereof.

Exemplary binding agents include, but are not limited to, starch (e.g., corn-starch and starch paste); gelatin; sugars (e.g., sucrose, glucose, dextrose, dextrin, molasses, lactose, lactitol, mannitol); natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone), magnesium aluminium silicate (Veegum), and larch arabogalactan); alginates; polyethylene oxide; polyethylene glycol; inorganic calcium salts; silicic acid; polymethacrylates; waxes; water; alcohol; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, ascorbyl palmitate, ascorbyl stearate, ascorbyl oleate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and combinations thereof.

The preparation of all these pharmaceutical compositions is according to the common practices known by the skilled person in the art.

When the hybrid promoter, construct, or cell of the invention include a gene operatively linked to the promoter, said gene being a gene associated to a disease, for example when mutated, all the previous aspects of the invention and their respective embodiments can be used as a medicament, allowing the expression of a functional protein from said gene operatively linked to the hybrid promoter. Thus, in a fifth aspect, the invention relates to a promoter as defined in the first aspect, to a polynucleotide construct as defined in the second aspect, to a vector as defined in the third aspect or to a cell as defined in the fourth aspect, or a pharmaceutical composition comprising one or more of them, for use as a medicament.

In a more particular embodiment, hybrid promoter, the polynucleotide construct, the vector, the cell or the pharmaceutical composition for use according to the previous aspect, the medicament is for use in the prevention and/or treatment of a kidney disease.

This embodiment can also be formulated as the use of hybrid promoter, the polynucleotide construct, the vector, the cell or the pharmaceutical composition as defined above for the manufacture of a medicament for the prevention and/or treatment of a kidney disease. The present invention also relates to a method for the treatment and/or prevention of a kidney disease, comprising administering a therapeutically effective amount of an hybrid promoter, a polynucleotide construct, a vector, ta cell or a pharmaceutical composition as defined above, together with pharmaceutically acceptable excipients or carriers, in a subject in need thereof, including a human.

In a more particular embodiment, the kidney disease is selected from one or more of glomerular genetic disorders including autosomal recessive steroid-resistant nephrotic syndrome, autosomal dominant steroid-resistant nephrotic syndrome, Denys-Drash syndrome, Frasier's syndrome, WAGR (Wilms' tumour, aniridia, genitourinary anomalies, retardation) syndrome, Pierson's syndrome, Nail-patella syndrome, Schimke immuno-osseous dystrophy, mitochondrial disorders with steroid resistant nephrotic syndrome, Fabry's disease, Alport's syndrome, benign familial haematuria (thin basement membrane nephropathy), Fechtner's syndrome (Alport's syndrome with macrothrombocytopenia), Alport's syndrome with leiomyomatosis and familial amyloidosis and other renal disorders , such as IgA nephropathy, diabetic nephropathy and Dent disease.

The hybrid promoter, polynucleotide construct, vector, cell or pharmaceutical composition according to the present invention are for use to be administered parenterally, for example, intravenously, or by infusion techniques. One or more of them are administered, in a particular embodiment, in the form of a sterile aqueous solution which contains, for example, other substances, salts or glucose to make the solution isotonic with blood. The aqueous is, in particular, buffered (preferably to a pH of from 3 to 9). The pharmaceutical composition may be formulated accordingly. The preparation of suitable parenteral formulations under sterile conditions is readily accomplished by standard pharmaceutical techniques well-known to those skilled in the art. The hybrid promoter, polynucleotide construct, vector, cell or pharmaceutical composition according to the present invention are for the indicated used and adapted to be administered systemically, such as by intravenous injection.

The hybrid promoter, polynucleotide construct, vector, cell or pharmaceutical composition according to the present invention are, in another particular embodiment, adapted for the indicated used and to be administered locally, for example by targeting administration to the kidney. Suitably, they are for use to be administered by injection into the renal artery, or renal vein, or by ureteral or subcapsular injection. In embodiments of the invention the hybrid promoter, polynucleotide construct, vector, cell or pharmaceutical composition are to be administered by injection into the renal artery. In alternative embodiments of the invention, they are to be administered by retrograde administration, e.g., via the ureters using a urinary catheter.

For the particular administration to the renal vein in mice, inventors propose a method that allows the administration of low volumes (around 50 microlitres). This low administration avoids cell damage in kidney.

The particular method of administration comprising the clamping of the hilum and the ureter. Then around 50 µl of a composition comprising the vector of the invention, or the polynucleotide construct, or the cell of the invention, or any pharmaceutical composition with one or more of them, is injected to the renal vein. In particular it is retrogradely injected into renal vein. Particular doses in mice are from 5*10E10 vector genomes (vg)/mouse to 5*10E11 vg/mouse. These doses can be transferred to human beings according to scales for equivalency.

By this mode of administration, the inventors could administer a reporter gene of interest using a polynucleotide construct with a constitutive promoter (cytomegalovirus) as a reference example. The volume of injection usually around 100 µl was reduced to 50 µl as indicated. Moreover, despite the high pressure impaired to kidney tissues, due to the low but effective dose (i.e., volume), no cell damage was observed (data not shown). The inventors detected that by this mode of administration and with the constitutive promoter, expression took also effect in the liver. This proves that by this mode of administration the injected construct, vector, cell, etc., enters systemic circulation. This is noteworthy, because as will also be demonstrated in example below, the constructs, vectors, or cells with the hybrid promoter of the invention were highly selective for podocytes, being express in liver cells at minor extent that other constructs of the prior art. Therefore, the proposed promoter and derivative polynucleotide constructs, vectors or cells are safe in terms of their tissue expression in the desired targets, when systemically administered. Moreover, if a particular polyadenylation signal is in the construct accompanying the hybrid promoter, expression in the diseased cells is enhanced.

The hybrid promoter, polynucleotide construct, vector, cell or pharmaceutical composition are, in a particular embodiment, to be administered as a single dose, in other words, subsequent doses are not needed. In the event that repeated doses are needed different or the same AAVs serotypes are used in a particular embodiment.

Optionally hybrid promoter, polynucleotide construct, vector, cell or pharmaceutical composition are to be administered in combination with other therapeutic approaches, such as a in particular temporary immunosuppressive therapy. Other therapeutic approaches to be considered in combination with the hybrid promoter, polynucleotide construct, vector, cell or pharmaceutical composition may include inhibition of the renin-angiotensin-aldosterone system (RAAS) , using agents such as angiotensin converting enzyme inhibitors (ACEi), angiotensin receptor blockers (ARBs), or aldosterone antagonist compounds (e.g., spironolactone, and new agents as firenenone) , or even in near future, potentially, new promising therapies as spasartan, sodium-glucose transport protein 2 (SGLT2) inhibitors, or other approved compounds to decrease proteinuria and progression to chronic kidney disease (CKD).

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Example 1. Expression of GFP by constructs with the podocyte-specific hybrid promoter of the invention and comparison with constructs of prior art with other podocyte-specific promoters

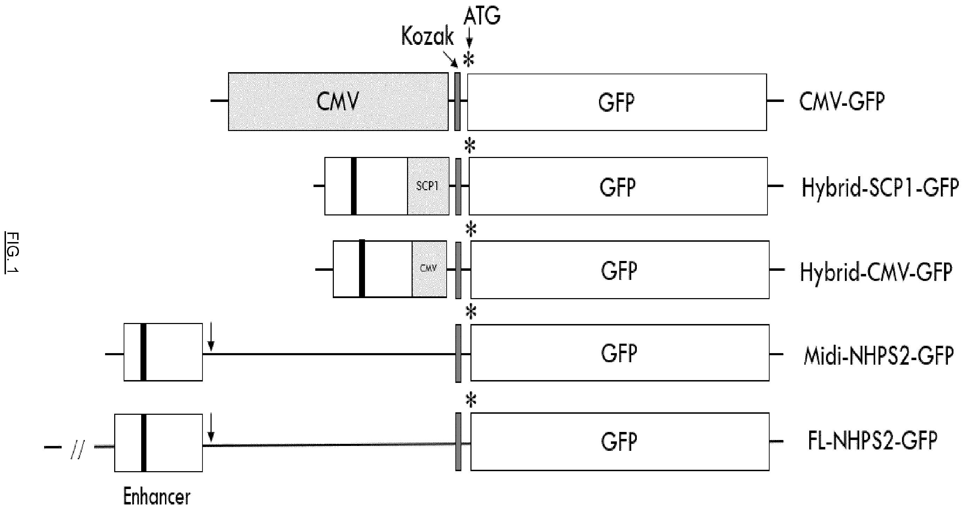

The polynucleotide construct defined by SEQ ID NO: 10 (also termed in this description as 0.3NPHS2-SCP1_KGFP_WPRE3_minSV40pA, or Hybrid-SCP1-GFP in FIG. 1), including the podocyte-specific hybrid promoter of SEQ ID NO: 1 to express the reporter green fluorescent protein was expressed by insertion into plasmids in podocytes, (E11 cell line), HEK293 cells (ATCC #CRL-11268). HepG2 cells (any commercially available), HeLa (#CCL-2) and Renal Proximal Tubule Cells _RPTEC cells (any commercially available). The same experiment was carried out with SEQ ID NO: 11 (also termed in this description 0.2NPHS2-minCMV_KTurboGFP_WPRE3_minSV40pA or hybrid-CMV-GFP in FIG. 1)

As comparative examples, the same (plasmids) were loaded with the polynucleotide constructs defined by SEQ ID NO: 14 and SEQ ID NO: 15, and which correspond respectively to the constructs assayed by Harendza et al. 2009 and Moeller et al. 2003 , both including a promoter different from the one defined by SEQ ID NO: 1. SEQ ID NO: 14 is also termed in this description as 0.8NPHS2_KGFP_WPRE3_minSV40pA or Midi-NHPS2-GFP in FIG. 1. SEQ ID NO: 15 is also termed in this description as 2.5NPHS2_KGFP_WPRE3_minSV40pA or FL-NHPS2-GFP in FIG. 1)

As control, SEQ ID NO: 16 (also termed CMV_KGFP_WPRE3_minSV40pA or in FIG 1 as CMV-GFP).

Cells were transfected through transitory transfection using Lipofectamine 3000 (Thermo Fisher #L3000008). Cells were cultured in DMEM (Gibco #42430082) supplemented with 10% FBS (Gibco #10270098), 1% sodium pyruvate [100 mM] (Gibco #11360039), 1% antibiotic/antimycotic (Gibco #15240-062) and 0.2% plasmocin (Invivogen #ant-mpp) at 37°C with 5% CO2., T^{a}, RH, culture media etc.) and Expression was determined at 48 hours post-transfection.

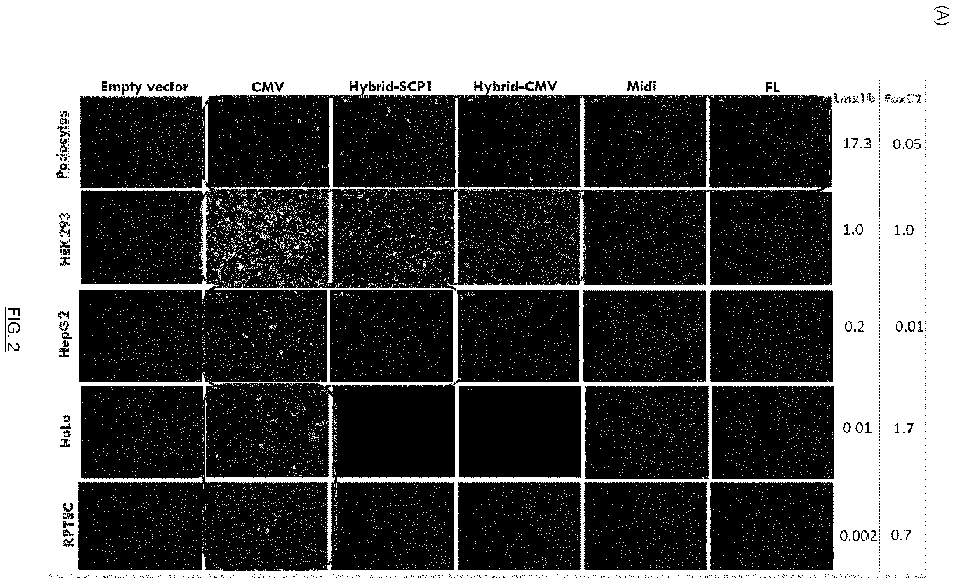

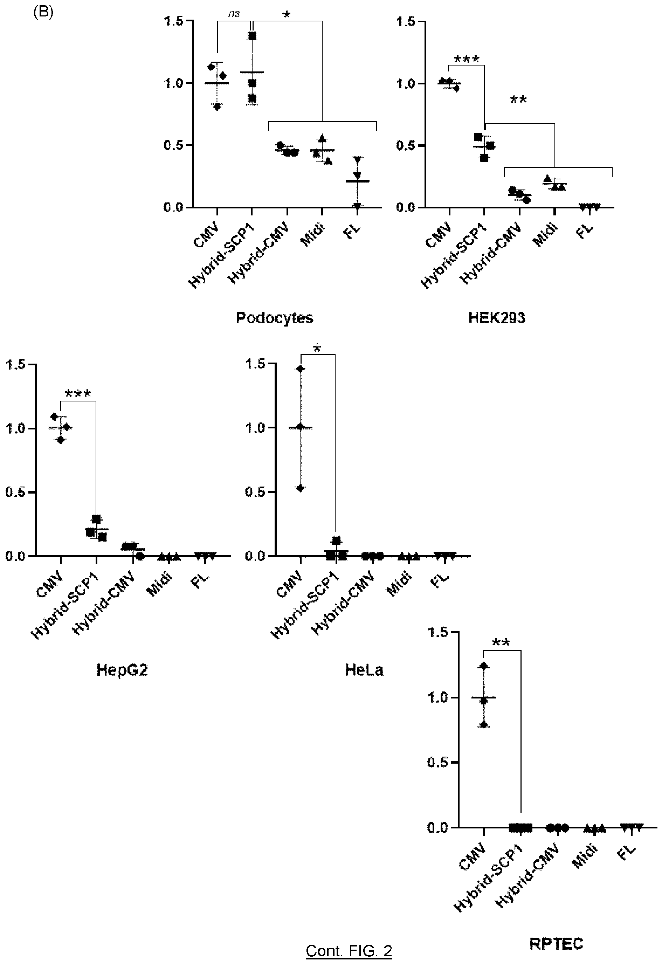

The fluorescence in the cells, measured as green fluorescent cells, was detected by microscope. A fluorescent microscope confocal image of one replica of the assay is illustrated in FIG. 2 (A). In FIG. 2 (B) the graphics show the relative fluorescence of the cells per field (Rel GFP cell/field in each of the cell types assayed.

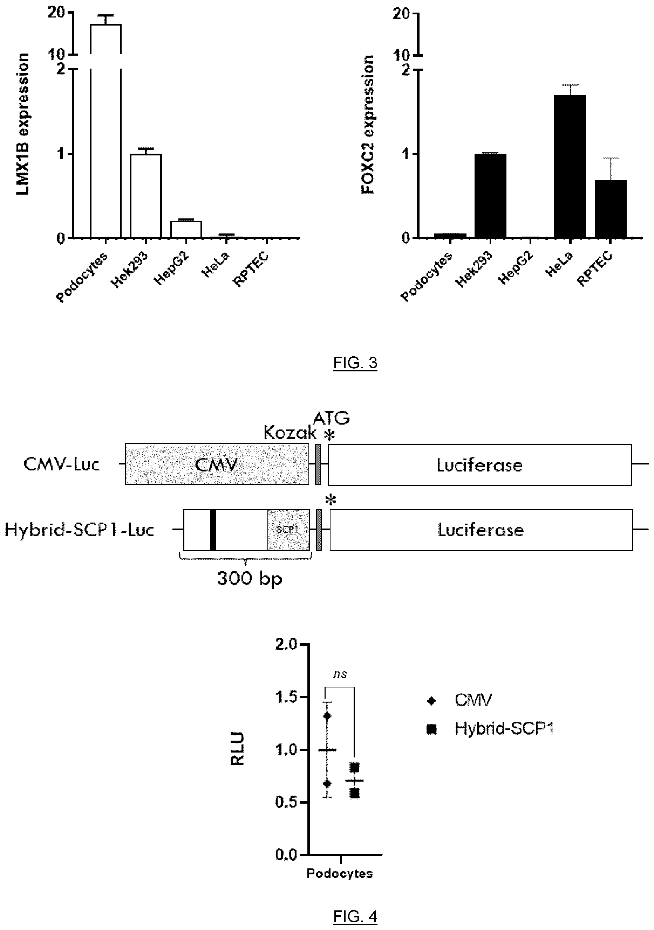

Finally, in FIG. 3 there is depicted the expression of Lmx1b and Foxc2 transcription factors in the selected cell lines.

Data from this Example allow affirming that higher expression in podocytes took place with the constructs of the invention.

### Example 2. Confirmation of expression of the podocyte-specific hybrid promoter of the invention with Luciferase constructs

The promoter of the invention of SEQ ID NO. 1 was tested in a construct comprising as reporter gene the luciferase gene. The construct of SEQ ID NO: 8 included as polyadenylation signal the early SV40 polyadenylation signal.

The control in the assay was the construct of SEQ ID NO: 17 (CMV_Kluciferase_WPRE3_minSV40pA), including the constitutive CMV promoter.

Data are illustrated in FIG. 4, where a schematic view of the promoters and luciferase gene are depicted and the expression in podocytes is indicated as relative luciferase units (RLU) in the podocytes for each of the constructs. The assay was performed with plasmids as in Example 1.

### Example 3. Selectivity for diseased cells.

Inventors surprisingly found that when the polyadenylation signal in the construct of the invention was a 3'-UTR sequence comprising two CPE and a cytoplasmic polyadenylation signal of sequence AAUAAA (CPEpolyA), the expression was higher in maintained stress conditions (i.e., endoplasmic reticulum (ER) stress conditions).

This assay was carried out with a renal cell line (HEK293, ATCC #CRL-11268) and data demonstrate that in comparison with the polyadenylation signal known as SV40 poly A, the CPEpolyA allowed expression of luciferase gene in stress conditions, even at a higher expression after long time of stress. In contrast, the construct with SV40polyA maintained the expression all along time but at a lower extent.

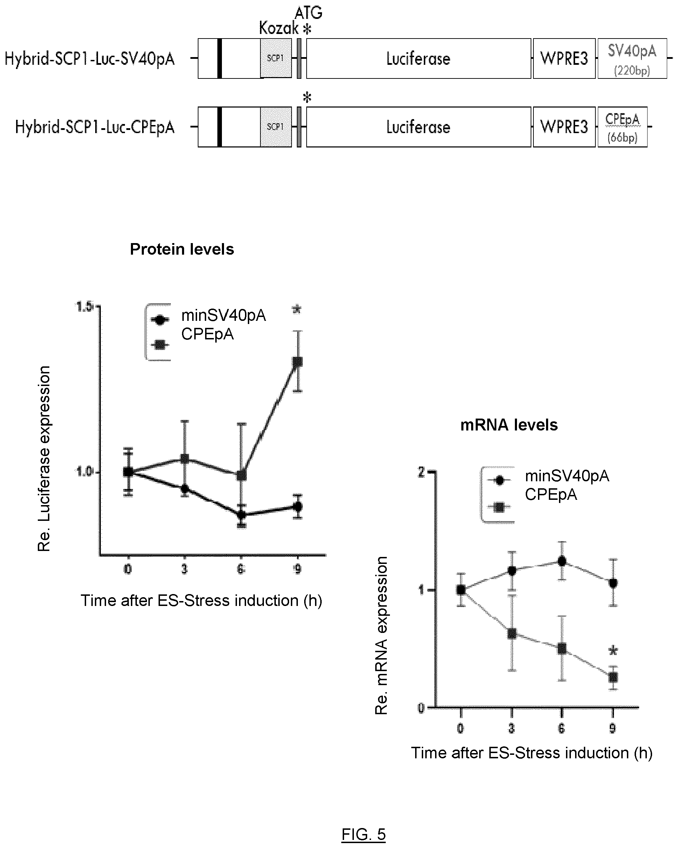

The two tested versions of constructs comprising the podocyte-specific promoter of SEQ ID NO: 1 used in these assay were the construct of SEQ ID NO: 8, including as polyadenylation signal the early SV40 polyadenylation signal (SV40poly A in FIG. 5); and the construct of SEQ ID NO: 9 (CPEpolyA in FIG. 5), that included as polyadenylation signal an untranslated sequence in 3' position (3'-UTR) with respect to the luciferase gene and including two cytoplasmic polyadenylation elements (CPE).

The scheme of the elements integrating in the constructs is also illustrated in FIG. 5. This figure illustrates the expression under endoplasmic reticulum stress conditions (ER stress) for each of them. The figure includes the data of protein expressions (relative luciferase expression) and mRNA levels (relative mRNA expression) are provided along time of ER stress. The skilled person in the art will know how to induce ER stress conditions and how to calculate relative expressions of the protein and mRNA levels.

These data show, thus a synergism when certain elements in the polynucleotide construct of the invention are combined with the podocyte-specific hybrid promoter.

### Example 4. Expression of CRB2 protein in HEK293 cells

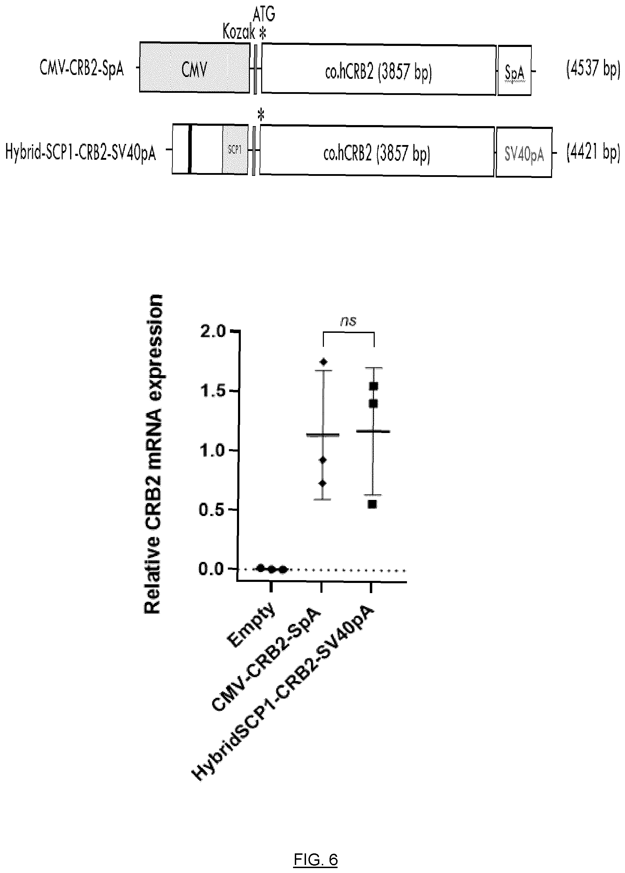

The constructs of SEQ ID NO: 18 comprising the podocyte-specific hybrid promoter of SEQ ID NO: 1 and the gene of CRB2 (cohCRB2) was used for the plasmid transfection of HEK293 cells. SEQ ID NO: 18 (as Hybrid-SCP1-CRB2-SV40pA in FIG. 6) included as polyadenylation signal the early SV40 polyadenylation signal.

As comparative example, the construct of SEQ ID NO: 5 with the constitutive cytomegalovirus promoter and the CRB2 gene was also transfected. SEQ ID NO: 5 includes as polyadenylation signal the one known as synthetic polyadenylation signal (SpA). An empty vector was used as control.

Data of this assay are depicted in FIG. 6, which shows that using the promoter of SEQ ID NO: 1 (or one with 85% of identity) the expression of Crb2 gene (a gene of 3.8 kb) was in the ratio of the expression using the constitutive CMV promoter, which is known as a strong promoter.

### Citation List

### Patent Literature

- WO2020148548A1 (The University of Bristol)
- WO2021181118A1 (The University of Bristol et al.)
- WO2017153606 (Fundació Institut de Recerca Biomèdica (IRB Barcelona), et al.)

### Non Patent Literature

- Pique et al in "A Combinatorial Code for CPE-Mediated Translational Control" Cell 2008, 132(3): 434-48.
- Juven-Gershon et al., "rational design of super core promoter that enhances gene expression", Nature Methods-2006, vol. no. 3(11), pp. 917-922.
- Needleman and Wunsch, 1970, J. Mol. Biol. 48: 443-453
- EMBOSS: The European Molecular Biology Open Software Suite, Rice et al., 2000, Trends Genet. 16: 276-277
- Altschul et al., "Basic local alignment search tool", 1990, J. Mol. Biol, v. 215, pages 403-410.
- Harendza et al. 2009
- Moeller et al. 2003

## Claims

1. A podocyte-specific hybrid promoter which comprises:
(a) a podocyte-specific enhancer sequence comprising SEQ ID NO: 13 (AAAAACAGAAGTTAGACCAGACCCCTTCCTGCCTATGATTCTTCAAGAAGCATTGCA TCATCAACATCAGGCATAAGCATTAATAAAGACCCTAAATAATAACAGAGACGAAACA CATCGCAAAGAGAGTTTTCTTTTATCCCCTTTAAAATGTAAATACTCCCAGGAGGAAT CAGCCAACATCATTAGGGGTTAATGCATATG), or a sequence with a percentage of identity with SEQ ID NO: 13 of at least 85%; and
(b) a core promoter sequence selected from:
(b1) minimal core promoter of 81 nucleotides length with a percentage of identity from 85 to 100 % with SEQ ID NO: 2 (GTACTTATATAAGGGGGTGGGGGCGCGTTCGTCCTCAGTCGCGATCGAACACTCGA GCCGAGCAGACGTGCCTACGGACCG, and
(b2) minimal core promoter of 56 nucleotides length with a percentage of identity from 85 to 100 % with SEQ ID NO: 20 (GGCGTTTACTATGGGAGGTCTATATAAGCAGAGCTCGTTTAGTGAACCGTCAGATC).

2. The podocyte-specific hybrid promoter according to claim 1, which consists in the polynucleotide sequence SEQ ID NO: 1, or in a nucleotide sequence with a percentage of identity of at least 85 % with said SEQ ID NO: 1

(AAAAACAGAAGTTAGACCAGACCCCTTCCTGCCTATGATTCTTCAAGAAGCATTGCA
TCATCAACATCAGGCATAAGCATTAATAAAGACCCTAAATAATAACAGAGACGAAACA
CATCGCAAAGAGAGTTTTCTTTTATCCCCTTTAAAATGTAAATACTCCCAGGAGGAAT
CAGCCAACATCATTAGGGGTTAATGCATATGTAGAATAACTAGGGCGTACTTATATAA
GGGGGTGGGGGCGCGTTCGTCCTCAGTCGCGATCGAACACTCGAGCCGAGCAGAC
GTGCCTACGGACCG).

3. A polynucleotide construct comprising the isolated polynucleotide hybrid promoter as defined in any of claims 1-2, and a nucleotide sequence of a gene or a fragment of a gene of interest operatively linked to the said podocyte-specific hybrid promoter.

4. A polynucleotide construct according to claim 3, further comprising one or more of:
(a) a polyadenylation signal nucleotide sequence;
(b) a protein translation initiation site consensus nucleotide sequence;
(c) a post-transcriptional regulatory element nucleotide sequence; and
(d) 5' and 3' inverted terminal repeat nucleotide sequences.

5. The polynucleotide construct according to any one of claims 3 or 4, comprising in the 5' to 3' direction:
(i) a 5' inverted terminal repeat nucleotide sequence;
(ii) the isolated polynucleotide hybrid promoter as defined in any of claims 1-2;
(iii) a protein translation initiation site consensus nucleotide sequence;
(iv) a nucleotide sequence of a gene or a fragment of a gene of interest;
(v) a post-transcriptional regulatory element nucleotide sequence;
(vi) a polyadenylation signal nucleotide sequence; and
(vii) a 3' inverted terminal repeat nucleotide sequence.

6. The polynucleotide construct according to any one of claims 3-5, wherein the protein translation initiation site consensus nucleotide sequence is the kozak consensus sequence.

7. The polynucleotide construct according to any one of claims 3-6, wherein the post-transcriptional regulatory element nucleotide sequence is a Woodchuck hepatitis postranscription regulatory element (WPRE).

8. The polynucleotide construct according to any one of claims 3-7, wherein the polyadenylation signal nucleotide sequence is selected from bovine growth hormone (bGH) polyadenylation signal, early SV40 polyadenylation signal, synthetic polyadenylation signal (SpA), and an untranslated sequence in 3' position (3'-UTR) with respect to the polynucleotide sequence of a gene or a fragment of a gene of interest that comprises at least two cytoplasmic polyadenylation elements which are separated by less than 50 nucleotides, and a cytoplasmic polyadenylation signal which is separated by less than 100 nucleotides from the first or second cytoplasmic polyadenylation element.

9. The polynucleotide construct according to any one of claims 3-8, wherein the gene of interest operatively linked to the podocyte-specific hybrid promoter is selected from:
- a gene associated with one or more kidney diseases, and/or
- a gene with a length up to 4.0-5.0 kilobases (kb).

10. A vector comprising the podocyte-specific hybrid promoter as defined in any of claims 1 or 2, or the polynucleotide construct as defined in any one of claims 3-9.

11. The vector according to claim 10, which is an adeno-associated virus vector (AAV), with tropism to renal cells and podocytes, in particular selected from one or more of the serotypes AAV19, AAV2, AAV3B, AAV4, AAV8, AAV9, AAVrh10, AAV-LK03, AAV-DJ, and AAV-DJ/8.

12. A cell comprising the podocyte-specific hybrid promoter as defined in any of claims 1 or 2, or the polynucleotide construct according to any of claims 3-9, or the vector according to any of claims 10-11.

13. A pharmaceutical composition comprising a therapeutically effective amount of the polynucleotide construct according to any of claims 3-9, the vector according to any of claims 10- 11 or the cell according to claim 12, together with one or more pharmaceutically acceptable excipients and/or carriers.

14. A polynucleotide construct as defined in any of claims 3-9, a vector as defined in any of claims 10- 11, or the cell as defined in claim 12, or the pharmaceutical composition as defined in claim 13, for use as a medicament.

15. The polynucleotide construct, the vector, the cell or the pharmaceutical composition for use according to claim 14, which is for use in the prevention and/or treatment of a kidney disease.
